(19) Europäisches Patentamt
European Patent Office
Office européen des brevets

(11) Veröffentlichungsnummer: **0 008 031**
**B1**

(12) # EUROPÄISCHE PATENTSCHRIFT

(45) Veröffentlichungstag der Patentschrift: **29.02.84**

(51) Int. Cl.³: **C 12 P 19/26**

(21) Anmeldenummer: **79102600.8**

(22) Anmeldetag: **23.07.79**

(54) **Verfahren zur Herstellung von 6-Amino-6-desoxy-L-sorbose.**

(30) Priorität: **03.08.78 DE 2834122**

(43) Veröffentlichungstag der Anmeldung:
**20.02.80 Patentblatt 80/4**

(45) Bekanntmachung des Hinweises auf die Patenterteilung:
**29.02.84 Patentblatt 84/9**

(84) Benannte Vertragsstaaten:
**CH DE FR GB**

(56) Entgegenhaltungen:

**CHEMISCHE BERICHTE 100, 1967, DE H. PAULSEN: "Synthese und Reaktionen von Keto-peperidinosen" Zeilen 802-815**

(73) Patentinhaber: **BAYER AG**
**Zentralbereich Patente, Marken und Lizenzen**
**D-5090 Leverkusen 1, Bayerwerk (DE)**

(72) Erfinder: **Kinast, Günther, Dr.**
**Am Eckbusch 42**
**D-5600 Wuppertal 1 (DE)**
Erfinder: **Schedel, Michael, Dr.**
**Sillerstrasse 49**
**D-5600 Wuppertal 11 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 031 B1

Verfahren zur Herstellung von 6-Amino-6-desoxy-L-sorbose

Die vorliegende Erfindung betrifft ein neues, mikrobiologisches Verfahren zur Herstellung von 6-Amino-6-desoxy-L-sorbose aus 1-Amino-1-desoxy-D-glucitol unter Verwendung aerober Bakterien.

6-Amino-6-desoxy-L-sorbose der Formel I

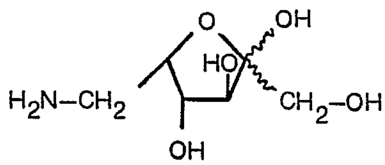

(I)

wurde bisher in einer 8-stufigan Synthese auf rein chemischem Wege aus Sorbose hergestellt (H. Paulsen, I. Sangster und H. Heins, Chem. Ber. *100,* 802 bis 815 (1967)).

6-Amino-6-desoxy-L-sorbose liegt in saurem Medium in der Furanose-Form (Ia), im alkalischen in der Piperidinose-Form (Ib), die sich mit der Verbindung (Ic) im Gleichgewicht befindet, vor.

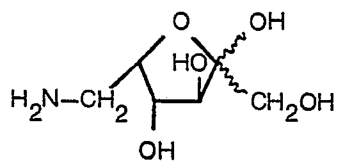

Ia

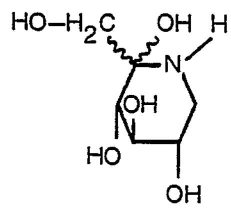

Ib

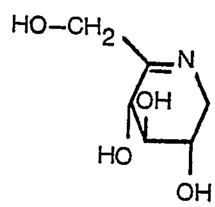

Ic

Die Verbindungen Ia, Ib und Ic sind sehr instabil und lagern sich in wäßriger Lösung, besonders im sauren pH-Bereich, durch Wasserabspaltung irreversibel in das Pyridinderivat der Formel II um.

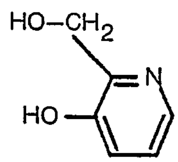

(II)

Est ist weiterhin bekannt (J. K. N. Jones, M. B. Perry und J. C. Turner, Can.J.Chem. *39,* 2400—2410 (1961)), daß sich 6-Desoxy-6-N-methylacetamido-L-sorbose aus 1-Desoxy-1-N-methylacetamido-D-glucitol durch 19-tägige mikrobiologische Oxidation mit Acetobacter suboxidans in 36%iger Ausbeute herstellen läßt. Die lange Reaktionsdauer und die Tatsache, daß die Aminogruppe durch Acetylierung geschützt werden muß, lassen aufgrund der bektannten chemischen Instablität von (I) dieses Verfahren wenig geeignet erscheinen zur Herstellung von L-sorbose (I).

Darüberhinaus ist die Verwendung von Acetobacter suboxidans bei mirkobiologischen Oxidationen von Zuckeralkoholen bekannt (vergl. Kieselich, Microbial transformations of non-steroid cyclic compounds (1976) Seiten 272—273).

Es wurde nun gefunden, daß man 6-Amino-6-desoxy-L-sorbose (I) erhält, wenn man 1-Amino-1-desoxy-D-glucitol mit Sauerstoff unter Einwirkung von aeroben oder fakultativ aeroben Mikroorganismen oder von deren Extrakten in geeigneten Medien und unter geeigneten Bedingungen gemäß der folgenden Reaktion oxidiert:

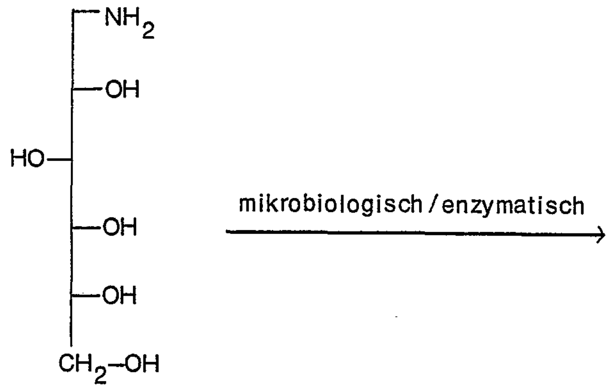


(I)

Die mikrobiologisch/enzymatische Oxidation von 1-Amino-1-desoxy-D-glucitol zu 6-Amino-6-desoxy-L-sorbose (I) ist unter günstigen Bedingungen bereits nach wenigen Stunden beendet. Eine Zersetzung von 6-Amino-6-desoxy-L-sorbose ist bei dieser kurzen Reaktionsdauer vernachlässigbar gering.

Das gewünschte Produkt kann als solches isoliert oder weiter umgesetzt werden, beispielsweise durch katalytische Hydrierung zu 1-Desoxy-nojirimycin (III)

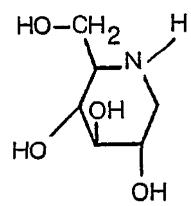

(III)

Est ist als ausgesprochen überraschend zu bezeichnen, daß man nach dem erfindungsgemäßen mikrobiologisch/enzymatischen Verfahren 6-Amino-6-desoxy-L-sorbose auf einfache Weise in kurzer Zeit und hoher Ausbeute erhält, denn im Hinblick auf den Stand der Technik war zu erwarten, daß man nur am Stickstoff, beispielsweise durch Acetyl geschützte 1-Amino-1-desoxy-D-glucitole mit Mikroorganismen zu den entsprechenden am Stickstoff geschützten Amino-L-sorbosen oxidieren und anschließend isoliern kann (J. K. N. Jones, M. B. Perry und J. C. Turner, Can.J.Chem. *39*, 2400—2410 (1961)), weil 6-Amino-6-desoxy-L-sorbose in wäßrigem Medium im angewandten pH-Bereich bei der beschriebenen mehrtägigen Fermentationsdauer zum unerwünschten Pyridinderivat (II) weiter reagiert.

Das beim erfindungsgemäßen Verfahren eingesetzte Ausgangsmaterial 1-Amino-1-desoxy-D-glucitol ist bekannt; es läßt sich beispielsweise aus D-Glucose durch reduktive Aminierung mit Ammoniak, Wasserstoff und Nickel als Katalysator herstellen. Das Ausgangsprodukt kann als solches oder als Salz, beispielsweise als Chlorid, Sulfat, Nitrat, Acetat, Oxalat oder Dihydrogenphosphat eingesetzt werden.

Mikroorganismen, die zur Durchführung des erfindungsgemäßen Verfahren geeignet sind oder aus denen aktive Extrakte zur Durchführung des erfindungsgemäßen Verfahrens gewonnen werden können, können Prokaryonten also Bakterien oder Eukaryonten, z.B. Pilze, sein und jeweils den verschiedensten taxonomischen Gruppen angehören. Der Fachmann auf mikrobiolgischem Gebiet kann geeigente Mikroorganismen leicht finden, indem er eine größere Zahl aerober ode fakultativ aerober Mikroorganismen, wie sie. z.B. öffentliche Sammlungen zur Verfügung stellen, in einem entsprechenden Nährmedium, das 1-Amino-1-desoxy-D-glucitol enthält, kultiviert und auf die Fähigkeit überprüft, die erfindungsgemäße Oxidationsreaktion zu katalysieren und 6-Amino-6-desoxy-L-sorbose anzuhäufen.

Indem nach dieser Vorschrift vorgegangen wurde, konnten als für das erfindungsgemäße Verfahren geeignete Mikroorganismen z.B. Bakterien der Ordnung Pseudomonadales, innerhalb dieser Ordnung besonders Vertreter der Familie Pseudomonadaceae und hier allen Dingen Bakterien der Gattung Gluconobacter gefunden werden. Ferner haben sich auch Bakterien aus der Gruppe der coryneformen Bakterien, besonders solche der Gattung Corynebacterium als geeignet erwiesen. Schließlich konnte das erfindungsgemäße Verfahren auch mit Pilzen, so z.B. mit Hefen oder Ordnung Endomycetales, besonders mit solchem der Familie Spermophthoraceae und hier hauptsächlich mit Vertretern der Gattung Metschnikowia durchgeführt werden.

Als Beispiele seien genannt:

Gluconobacter oxidans ssp. suboxydans (DSM 50 049), Glucobacter oxidans ssp. suboxydans (DSM 2003), Corynebacterium betae (DSM 20141) und Metschnikowia pulcherrima (ATCC 20 515).

Die mit DSM-Nummern angegebenen Mikroorganismen befinden sich in der Deutschen Sammlung für Mikroorganismen, Göttingen und sind jedermann frei zugänglich. Metschnikowia

pulcherrima ist bei der American Type Culture Collection, Rockville, Maryland, USA unter der angegebenen Nummber am 21.7.1978 hinterlegt worden.

Wird das erfindungsgemäße Verfahren mit intakten Mikroorganismen in wachsender Kultur durchgeführt, können feste, halbfeste oder flüssige Nährmedien verwandt werden. Bevorzugt werden wäßrig-flüssige Nährmedien herangezogen.

Die Kultur kann in allen Nährmedien durchgeführt werden, welche bekannterweise zur Kultivierung von Mikroorganismen der obengenannten Gruppen verwendet werden und die des im erfindungsgemäßen Verfahren zu oxidierende 1-Amino-1-desoxy-D-glucitol enthalten. Das Nährmedium muß assimilierbare Kohlenstoff- und Stickstoffquellen, sowie Mineralsalze enthalten. Als assimilierbare Kohlenstoff- und Stickstoffquellen eignen sich vor allem komplexe Gemische wie sie insbesondere biologische Produkte verschiedenen Ursprungs darstellen, z.B. Sojabohnenmehl, Baumwollsamenmehl, Lisenmehl, Erbsenmehl, lösliche und unlösliche pflanzliche Proteine, Maisquellwasser, Hefeextrakt, Peptone und Fleischextrakt. Also Stickstoffquellen kommen zusätzlich Ammoniumsalze und Nitrate, z.B. Ammoniumchlorid, Ammoniumsulfat, Natriumnitrat und Kaliumnitrat, infrage. Die Mineralsalze, welche im Nährmedium enthalten sein sollen, liefern z.B. folgende Ionen; $Mg^{++}$, $Na^+$, $K^+$, $Ca^{++}$, $NH_4^+$, $Cl^-$, $SO_4^{--}$, $PO_4^{---}$ und $NO_3^-$ sowie Ionen der üblichen Spurenelemente wie Cu, Fe, Mn, Mo, Zn, Co und Ni.

Falls in den genannten komplexen Nährbodenbestandteilen oder im verwendeten Wasser diese Salze bzw. Spurenelemente nicht ausreichend vorhanden sind, ist es zweckmäßig, das Nährmedium entsprechend zu ergänzen.

Das im erfindungsgemäßen Verfahren zu oxidierende 1-Amino-1-desoxy-D-glucitol kann entweder allein oder im Gemisch mit einer oder mehreren oxidierbaren Verbindungen dem Grundnährmedium zugesetzt werden. Als zusätzliche oxidierbare Verbindungen können primäre Alkohole, beispielsweise Äthanol, sekundäre Alkohole, beispielsweise Isopropanol, Polyole, beispielsweise Sorbit oder Glycerin, Aldehyde, beispielsweise Glykolaldehyd, Aldosen, beispielsweise Glucose, oder Gluconsäuren verwendet werden.

Wird der Nährlösung eine oder mehrere der genannten Verbindungen zugesetzt, kann das zu oxidierende 1-Amino-1-desoxy-D-glucitol entweder vor dem Animpfen oder an einem beliebigen späteren Zeitpunkt zwischen früher lag-Phase und später stationärer Wachstumsphase zugesetzt werden. In einem solchen Fall wird der betreffende Organismus auf den jeweils zugesetzten oxidierbaren Verbindungen vorkultiviert.

Für das erfindungsgemäße Verfahren ist ein pH-Bereich zwischen 2 und 10 geeignet. Es ist günstig, die Kultur in diesem Bereich zu puffern, beispielsweise mit Phosphatoder Acetatpuffer.

Wie in der Fermentationstechnologie üblich, kann auch eine automatische pH-Regulierung durchgeführt werden, bei der sterile organische oder anorganische Säure, beispielsweise Schwefelsäure, oder sterile Lauge, beispielsweise Natronlauge, in Abständen in die Kulturlösung eingespritzt wird.

Wie allgemein bei mikrobiologischen Verfahren sollten Fremdinfektionen der Kulturmedien vermieden werden. Hierzu werden die üblichen Vorkehrungen getroffen, wie Sterilisation der Nährmedien, der Kulturgefäße sowie der für die Belüftung notwendigen Luft. Zur Sterilisation der Kulturgefäße können z.B. die Dampf- als auch die Trockensterilisation angewendet werden; Luft und die Kulturmedien können ebenfalls durch Dampf, aber auch durch Filtration sterilisiert werden.

Die Beimpfung der Nährmedien erfolgt nach allgemein üblichen Methoden, z.B. über Schrägröhrchen- oder Kolbenkulturen.

Die Kultur erfolgt unter aeroben Bedingungen und kann gemäß den allgemein üblichen Methoden, beispielsweise unter Verwendung von Schüttelkulturen z.B. in Schüttelkolben, von luftbewegten Kulturen oder von Submerskulturen durchgeführt werden. Bevorzugt erfolgt die Kultivierung im aeroben Submersverfahren in belüfteten Fermentern z.B. in üblichen Submersfermentationstanks. Es ist möglich, die Kultur kontinuierlich oder diskontinuierlich durchzuführen. Vorzugsweise wird diskontinuierlich gearbeitet.

Es ist zweckmäßige, sicherzustellen, daß die Mikroorganismen ausreichend mit Sauerstoff sowie den Nährstoffen in Kontakt gebracht werden. Dies kann nach der allgemein üblichen Methode wie Schütteln und Rühren, erfolgen.

Falls bei der Kultivierung in unerwünschter Menge Schaum entsteht, können die üblichen chemischen Schaumdämpfungsmittel, z.B. flüssige Fette und Öle, Öl-Wasser-Emulsionen, Paraffine, höhere Alkohole, wie Octadecanol, Siliconöle, Polyoxiäthylen- bzw. Polyoxipropylen-Verbindungen zugesetzt werden. Schaum kann auch mit Hilfe der üblichen mechanischen Vorrichtungen gedämpft oder beseitigt werden.

Die Züchtungstemperatur kann zwischen etwa 20 und etwa 45°C liegen. Die Dauer der Züchtung kann stark variiert werden, wobei z.B. die Zusammensetzung des Nährmediums und die Züchtungstemperatur eine Rolle spielen.

Die jeweiligen optimalen Bedingungen können von jedem Fachmann auf dem mikrobiologischen Gebiet leicht festgelegt werden.

Es hat sich herausgestellt, daß die Menge der sich in der Kulturbrühe anreichernden 6-Amino-6-desoxy-L-sorbose im allgemeinen ihr Maximum zwischen 5 Stunden und 5 Tagen nach Zugabe von 1-Amino-1-desoxy-D-glucitol erreicht.

Est ist auch möglich, die erfindungsgemäße Oxidationsreaktion mit konzentrierten Zellsuspensionen geeigneter Mikroorganismen durchzuführen. Konzentrierte Zellsuspensionen werden wie folgt hergestellt: Die in Frage kommenden Mikroorganismen werden in einer geeigneten Nährlösung kultiviert, dann z.B. durch Zentrifugation geerntet und in einem kleineren Volumen derselben Nährlösung oder in Salz- oder Pufferlösungen, z.B. physiologische Kochsalzlösung, wäßrigen Lösungen von $KH_2PO_4$, Na-Acetat, Maleinat oder einfach in Leitungs- oder destilliertem Wasser, suspendiert. Einer solchen Zellsuspension wird dann 1-Amino-1-desoxy-D-glucitol zugesetzt und die erfindungsgemäße Oxidationsreaktion unter den oben für wachsende Kulturen beschriebenen Bedingungen durchgeführt.

Der Vorteil dieses Verfahrens ist die durch die höhere Mikroorganismen-Konzentration ermöglichte Verkürzung der Reaktionsdauer des erfindungsgemäßen Verfahrens auf wenige Stunden.

Es ist weiterhin möglich, das erfindungsgemäße Verfahren nicht nur mit wachsenden Kulturen von Mikroorganismen oder mit daurus gewonnenen konzentrierten Zellsuspensionen durchzuführen, sondern auch mit aus diesen Bakterien hergestellten Extrakten oder Extraktfraktionierungen. Dabei kann es sich um Rohextrakte handeln, wie sie durch herkömmlichen Aufschluß von Mikroorganismenzellen gewonnen werden. Als Aufschlußmethoden können dienen: Ultraschallbehandlung, Passage durch eine French-Druckzelle, Zerreiben mit Quarzsand, Inkubation mit lysierenden Enzymen, Antolyse oder merhfaches Einfrieren und Auftauen.

Werden nichtfraktionierte Rohextrakte zur Oxidation von 1-Amino-1-desoxy-D-glucitol zu 6-Amino-6-desoxy-L-sorbose verwandt, haben sich prinzipiell dieselben Reaktionsbedingungen als güstig erwiesen, wie sie für die Durchführung des erfindungsgemäßen Verfahrens mit washsenden oder ruhenden Mikroorganismenzellen beschrieben wurden.

Soll das erfindungsgemäße Verfahren mit teilweise gereinigten Extraktpräparationen (Enzymen) durchgeführt werden, so können zur Gewinnung derartiger Präparationen die allgemein üblichen Methoden der Proteinchemie angewandt werden, wie Ultrazentrifugation, Fällungsreaktion, Ionenaustausch- oder Adsorptionschromatographie, Gelfiltration oder elektrophoretische Methoden. Zur Klärung der Frage, welche von hehreren durch eine der geannten Methoden gewonnenen Fraktionen zur Katalyse der erfindungsgemäßen Oxidationsreaktion geeignet ist, wird ein Aliquot dieser Fraktion bei einer Temperatur zwischen 20 und 45°C und einem pH zwischen 2 und 10 mit 1-Amino-1-desoxy-D-glucitol gemischt und der Ansatz auf die Bildung von 6-Amino-6-desoxy-L-sorbose anhand der Zunahme der Saccharase-Hemmaktivität (s. unten) untersucht. Zur Durchführung der erfindungsgemäßen Reaktion mit fraktionierten Zellextrakten kann es notwendig sein, daß zusätzlich Reaktionskomponenten dem Ansatz zugefügt werden müssen, wie z.B. physiologische oder künstliche Elektronenakzeptoren etwa $NAD^+$, $NADP^+$, Methylenblau, Dichlorphenolindophenol, Tetrazoliumsalze etc. Müssen solche zusätzlichen Reaktionskomponenten zugefügt werden, können diese entweder in Substratmengen, also in Konzentrationen, die der des eingesetzten 1-Amino-1-desoxy-D-glucitols entsprechen, oder in katalytischen Mengen, d.h. in Konzentrationen die deutlich unter der gewählten 1-Amino-1-desoxy-D-glucitol-Konzentration liegen, eingesetzt werden.

Soll im zweiten Fall eine annähernd quantitative Durchführung des erfindungsgemäßen Verfahrens gewährleistet sein, so ist dem Reaktionsansatz weiterhin ein System zuzufügen, das die nur in katalytischer Menge vorhandene Reaktionskomponente ständig regeneriert. Es kann sich dabei z.B. um ein Enzym handeln, das für die Reoxidation eines im Verlaufe der erfindungsgemäßen Reaktion reduzierten Elektronenakzeptors in Gegenwart von Sauerstoff oder anderen Oxisationsmitteln sorgt.

Ansonsten haben sich auch für die Durchführung des erfindungsgemäßen Verfahrens mit fraktionierten Zellextrakten dieselben Bedingungen als günstig erwiesen, wie sie oben für die Oxidation von 1-Amino-1-desoxy-D-glucitol zu 6-Amino-6-desoxy-L-sorbose in washsenden Mikroorganismenkulturen oder konzentrierten Zellsuspensionen angegeben wurden. Insbesondere gilt auch hier ein Temperaturbereich von 20 bis 45°C und ein pH-Bereich von 2 bis 10. Die Menge der gebildeten 6-Amino-6-desoxy-L-sorbose erreicht jedoch in einem kürzeren Zeitraum ihr Maximum. Je nach Extraktkonzentration genügt eine Inkubationszeit zwischen 2 Stunden und 3 Tagen.

Die zeitabhängige Bildung der 6-Amino-6-desoxy-L-sorbose im Kulturmedium kann entweder dünnschichtchromatographisch oder anhand der Zunahme der inhibitorischen Aktivität im Saccharase-Inhibitionstest, zweckmäßig mit der zweiten Methode, verfolgt werden.

Der Saccharase-Inhibitionstest in vitro ermöglicht die Bestimmung der enzyminhibitorischen Aktivität einer Substanz durch den Vergleich der Aktivität des solubilisierten intestinalen Disaccharidasen-Komplexes in Gegenwart bzw. in Absesenheit (sog. 100%-Wert) des Inhibitors. Als Substrat, welches die Spezifität des Inhibitortestes bestimmt, dient dabei eine praktisch glucose-freie Saccharose (Glucose <100 ppm), die Enzymaktivitätsbestimmung basiert auf der spektrophotometrischen Bestimmung freigesetzter Glucose mittels Glucosedehydrogenase und Nikotinamid-Adenin-Dinukleotid als Cofaktor.

Der intestinale Disaccharidasen-Komplex wird aus Schweinedünndarm-Mucosa durch tryptische Verdauung, Fällung aus 66%igem Äthanol bei −20°C, Aufnehmen des Präzipitates in 100 mM Phosphatpuffer (pH 7,0) und abschließende Dialyse gegen denselben Puffer gewonnen.

10 $\mu$l einer Probelösung, die so angesetzt ist, daß die Extinktion des Testansatzes mindestens 10%, jedoch nicht mehr als 25% unter der des 100%-Wertes liegt, werden mit 100 $\mu$l einer Verdünnung des intestinalen Disaccharidasen-Komplexes in 0,1 M Maleinat-Puffer (pH 6,25), versetzt und für

10 Minuten bei 37°C vorinkubiert. Die Verdünnung des Disaccharidasen-Komplexes ist auf eine Aktivität von 0,1 SE/ml einzustellen.

Eine Saccharase-Einheit (SE) ist als diejenige Enzymaktivität definiert, welche bei 37°C ein $\mu$mol Saccharose pro Minute spaltet und damit zur Freisetzung von je 1 $\mu$mol Glucose, welche im Test bestimmt wird, und Fructose, welche im Test nicht erfaßt wird, führt.

Anschließend wird die saccharolytische Reaktion durch Zugabe von 100 $\mu$l einer 0,4 M Lösung von Saccharose in 0,1 M Maleinat-Puffer (pH 6,25) gestartet und nach einer Inkubationsdauer von 20 Minuten bei 37°C durch die Zugabe von 1 ml Glucose-Dehydrogenase-Reagenz (Glucose-Dehydrogenase-Mutarotase-Gemisch, lyophyliert und 331,7 mg $\beta$-Nicotinamid-adenin-dinucleotit (freie Säure) in 250 ml 0,5 M Tris-Puffer pH 7,6, gelöst) abgestopp-t. Zum Nachweis der Glucose wird 30 Minuten bei 37°C inkubiert und anschließend bei 340 nm gegen einen Reagentienleerwert (mit Enzym, jedoch ohne Saccharose) fotometriert.

Un aus der im Saccharase-Inhibitionstest gefundenen Hemmung den Gehalt des erfindungsgemäßen Oxidationsproduktes 6-Amino-6-desoxy-L-sorbose im Kulturüberstand bestimmen zu können, wurden steigende Mengen reiner 6-Amino-6-desoxy-L-sorbose unter sonst identischen Bedingungen in den Saccharase-Inhibitionstest eingesetzt und die gefundene Hemmung in Prozent gegen die 6-Amino-6-desoxy-L-sorbose-Konzentration im Test aufgetragen. Es wurde eine hyperbolische Hemmkurve erhalten. Die Inhibitorkonzentration, die zu einer 50%igen Hemmung der Saccharase aus Schweinedünndarmmucosa führte, wurde aus dieser Eichkurve zu 1,2 $\mu$g 6-Amino-6-desoxy-L-sorbose/ml Testansatz bestimmt. Die Eichkurve wurde der bestimmung des Gehalts an 6-Amino-6-desoxy-L-sorbose im Kulturüber-stand zugrunde gelegt.

Die erfindungsgemäß erhaltene 6-Amino-6-desoxy-L-sorbose wird wie folgt aus der Kulturlösung gewonnen: Die Zellmesse wird abfiltriert oder abzentrifugiert und der Überstand über eine Säule mit saurem Ionenaustauscher gegeben und mit Wasser nachgewaschen. Dann wird mit 0,3 N Salzsäure eluiert und das Eluat eingeengt. Nach Zugabe von Äthanol kristallisiert das Hydrochlorid von 6-Amino-6-desoxy-L-sorbose aus. Die erfindungsgemäß erhaltene Verbindung hat die gleichen physikalischen Daten, die in der Literatur angegeben sind:

Schmelzpunkt: 127 bis 129°C (unter Zersetzung).

Sofern die erfindungsgemäß herzustellende Verbindung zu 1-Desoxy-nojirimycin weiter verarbeitet werden soll, kann auf die isolierung verzichtet werden. Hierzu wird die klare Lösung nach Entfernung der Zellmasse mit einem Katalysator wie Pd auf Kohle, Raney-Nickel oder $PtO_2$ und mit Wasserstoff unter einem Druck von 1 bis 80 bar hydriert. Nach Entfernen des Katalysators isoliert man 1-Desoxy-nojirimycink durch Chromatographbie an einem sauren Ionenaustauscher. Das so erhaltene 1-Desoxy-nojirimycin stimmt in allen physikalischen Eigenschaften mit authentischen Proben überein.

Nicht umgesetztes 1-Amino-1-desoxy-D-glucitol bzw. dessen Salze werden bei beiden Aufarbeitungsverfahren durch die Ionenaustauscher-Chromatographie wiedergewonnen und können für das erfindungsgemäße Verfahren als Ausgangsmaterial erneut verwendet werden.

## Beispiel 1

Oxidation von 1-Amino-1-desoxy-D-glucitol durch Glucobacter oxidans ssp. suboxidans in wachsender Kultur

Glucobacter oxidans ssp. suboxidans wurde in einer Flüssignährlösung vorkultiviert, die im Liter 20 g Hefeextrakt, 200 g Sorbit und 10 g $KH_2PO_4$ gelöst in entmineralisiertem Wasser enthielt. Die Nährlösung für die Vorkultur wurde auf einen pH-Wert von 5,0 eingestellt, zu je 100 ml in 1 l Erlenmeyerkolben gegeben und durch 15-minütiges Erhitzen im Auoklaven auf 121°C sterilisiert. Die Oxidation von 1-Amino-1-desoxy-D-glucitol wurde in einer Nährlösung durchgeführt, die pro Liter 20 g Hefeextrakt, 200 g Sorbit, 10 g $KH_2PO_4$ und 10 g·1-Amino-1-desoxy-D-glucitol (Oxalatsalz) gelöst in entmineralisiertem Wasser, enthielt, Der pH-Wert wurde mit verdünnter Salzsäure auf pH 5,0 eingestellt. Die Nährlösung wurde zu je 100 ml in 1 l Erlenmeyerkolben gegeben und durch 15-minütiges Erhitzen auf 121°C im Autoklaven sterilisiert. Nach dem Abkühlen wurde jeder Kolben mit jeweils 2 ml der Vorkultur beimpft und dann bei 28°C auf einer Schüttelmaschine bei 280 Umdrehungen pro Minute inkubiert. Die Bildung von 6-Amino-6-desoxy-L-sorbose wurde verfolgt, indem man eine entsprechend verdünnte Probe des Kulturüberstandes in Saccharase-Inhibitionstest untersuchte. Zu Zeitpunkt der höchsten Konzentration an 6-Amino-6-desoxy-L-sorbose (38 g/l nach 1 Tag, das entspricht 38% Umsatz) wurde die Fermentation abgebrochen. Die Kulturbrühen von 10 parallel durchgeführten Ansätzen wurden vereinigt, so daß zur Aufarbeitung ein Volumen von 1 l zur Verfügung stand.

Man trennte die durch Zentrifugieren ab, ließ das Filtrat durch eine Säule (40 cm lang, 2 cm Durchmesser), gefüllt mit stark saurem Ionenaustauscher Lewatit TSW 40, laufen, wusch mit Wasser nach und eluierte mit 0,3 normaler Salzsäure. Das Eluat wurde zu einem dünnflüssigen Sirup eingeent und mit Äthanol bis zur Trübung versetzt. Im Eisbad kristallisierte das Hydrochlorid von 6-Amino-6-desoxy-L-sorbose.

Schmelzpunkt: 127 bis 129°C (unter Zersetzung);

Hans Paulsen, Ian Sangster und Kurt Heyns, Chem. Ber. 100, 802—815 (1967):

Schmelzpunkt: 127 bis 129°C (unter Zersetzung).

Beispiel 2

Oxidation von 1-Amino-1-desoxy-D-glucitol durch Gluconobacter oxidans ssp. suboxydans in wachsender Kultur in Fermenter

Gluconobacter oxydans ssp. suboxydans wurde auf Schrägröhrchen vorkultiviert. Der Nährboden enthielt pro 1 l 10 g Hefeextrakt, 100 g Sorbit und 2 g Sorbit und 2 g $KH_2PO_4$, gelöst in Leitungswasser. Mit einem gut bewachsenen Rörchen wurde eine Flüssigkultur von 250 ml desselben Mediums jedoch ohne Agar in einem 1 Erlenmeyerkolben beimpft und bei 28°C auf einer Rundschüttelmaschine bei 280 Upm über Nacht inkubiert. Mit dieser 2, Vorkultur wurde ein 10 l Fermenter beimpft, der mit einem Flüssigmedium beschickt war, das pro 1 l 10 g Hefeextrakt, 100 g Sorbit, 2 g $KH_2PO_4$ und 10 g 1-Amino-1-desoxy-D-glucitol (HCl-Salz) enthielt. Der pH-Wert war auf 6,2 eingestellt worden. Das Medium war durch Autoklavieren bei 125°C für 60 Minuten sterilisiert worden. Der Fermenter wurde mit 5 l Luft pro Minute durchblasen und mit 500 Upm gerührt. Die Inkubationstemperatur war 28°C. Zu verschiedenen Zeiten wurden Proben steril entnommen und der Gehalt an 6-Amino-6-desoxy-L-sorbose anhand der Saccharase-Inhibitor-Aktivität bestimmt. Nach 1 1/2 Tagen waren 2,6 g/l 6-Amino-6-desoxy-L-sorbose vorhanden; das entsprach einem Umsatz von 26%. Aus der Kulturbrühe wurden die Zellen abzentrifugiert und der klare Überstand wie in beispiel 1 angegeben aufgearbeitet.

Beispiel 3

Oxidation von 1-Amino-1-desoxy-D-glucitol durch Corynebacterium betae in wachsender Kultur

Corynebacterium betae wurde auf Schrägröhrchen vorkultiviert, die folgenden Nährböden enthielten 10 g/l tryptisch verdautes Casein-Pepton, 6 g/l Hefeextrakt, 5 g/l Sorbit, 5 g/l NaCl, 20 g/l Agar in entmineralisiertem Wasser. Mit einem gut bewachsenen Schrägröhrchen wurden 250 ml Flüssignährlösung (im 1 l Erlenmeyerkolben) beimpft, die pro 1 l 10 g tryptisch verdautes Casein-Pepton, 5 g Hefeextrakt, 5 g Sorbit, 5 g NaCl und 2 g 1-Amino-1-desoxy-D-glucitol (Oxalat-Salz) enthielt. Die Nähbodenbestandteile wurden in entmineralisiertem Wasser gelöst und durch Autoklavieren bei 121°C für 20 Minuten sterilisiert. Die Kultur wurde bei 37°C auf einer Rungschüttelmaschine bei 200 Upm inkubiert. Der Gehalt an 6-Amino-6-desoxy-L-sorbose in der Kulturbrühe wurde anhand der Saccharose-Hemmaktivität verfolgt. Nach 1 1/2 Tagen waren 0,15 g/l vorhanden, das entsprach einem Umsatz von 7,5%. Zu diesem Zeitpunkt wurde die Fermentation abgebrochen, die Zellen durch zentrifugation abgetrennt und der klare Kulturüberstand wie in Beispiel 1 angegeben aufgearbeitet.

Beispiel 4

Oxidation von 1-Amino-1-desoxy-D-glucitol durch Metschnikowia pulcherrima in wachsender Kultur

Methschnikowia pulcherrima wurde in Schrägröhrchen auf einem Medium vorkultiviert, das pro 1 l 3 g Hefeextrakt, 6 g Pepton, 10 g Glucose, 8 g NaCl und 20 g Agar in entmineralisiertem Wasser enthielt. Mit einem gut gewachsenen Schrägröhrchen wurden 250 ml Flüssigmedium (in einem 1 l Erlenmeyerkolben) beimpft, das in 1 l 3 g Hefeextrakt, 6 g Pepton, 10 g Sorbit und 8 g NaCl gelöst in entmineralisiertem Wasser enthielt und durch Autoklavieren für 20 Minuten bei 121°C sterilisiert worden war. Die Kultur wurde bei 35°C auf einer Rundschüttelmaschine bei 200 Upm inkubiert. Der Gehalt an 6-Amino-6-desoxy-L-sorbose in der Kulturbrühe wurde anhand der Saccharase-Hemmaktivität verfolgt. Nach 2 Tagen waren 0,1 g/l vorhanden; das entsprach einem Umsatz von 5%. Die Fermentation wurde zu diesem Zeitpunkt abgebrochen, die Zellen durch Zentrifugation abgetrennt und der klare Kulturüberstand wie in Beispiel 1 angegeben aufgearbeitet.

Beispiel 5

Oxidation von 1-Amino-1-desoxy-D-glucitol durch eine konzentrierte Zellsuspension von Glucobacter oxydans ssp. suboxydans

Glucobacter oxydans ssp. suboxydans wurde im 10 l Maßstab im Fermenter in einem Medium angezogen, das pro 1 l 100 g Sorbit, 20 g Hefeextrakt und 2 g $KH_2PO_4$ — gelöst in Leitungswasser — enthielt. Der Fermenter wurde mit 5 l Luft pro Minute durchblasen, mit 500 Upm gerührt und bei 30°C temperiert. Nach 10-stündiger Inkubation wurden die Zellen aus der Kulturbrühe abzentrifugiert und in 1 l eines Mediums suspendiert, das 20 g Hefeextrakt, 2 g $KH_2PO_4$ und 10 g 1-Amino-1-desoxy-D-glucitol (Oxalatsalz) enthielt. Diese 10-fach konzentrierte Zellsuspension wurde in einem 1 l Fermenter bei 30°C inkubiert, mit 5 l Luft pro Minute durchblasen und mit 500 Upm gerührt. Nach 9 Stunden wurden die Zellen abzentrifugiert und der Überstand, der zu diesem Zeitpunkt 4,1 g/l 6-Amino-6-desoxy-L-sorbose — entsprechend einer 41%igen Umsetzung — enthielt, wie in Beispiel 1 angegeben aufgearbeitet.

Beispiel 6

Oxidation von 1-Amino-1-desoxy-D-glucitol durch einen zellfreien Extrakt von Glucobacter oxydans ssp. suboxydans

Glucobacter oxydans ssp. suboxydans wurde im 10 l Maßstab im Fermenter in einem Medium angezogen, das pro 1 l 100 g Sorbit, 20 g Hefeextrakt und 2 g $KG_2PO_4$ — gelöst in Leitungswasser — enthielt. Der Fermenter wurde mit 5 l Luft pro Minute durchblasen, mit 500 Upm gerührt und bei 30°C temperiert. Nach 16-stündiger Inkubation wurden die Zellen aus der Kulturbrühe abzentrifugiert, durch

Suspendieren in 10 mM $KH_2PO_4$ und erneutem Zentrifugieren einmal gewaschen und dann in 300 ml 10 mM $KH_2PO_4$ suspendiert. Die so erhaltene Zellsuspension wurde durch zweimalige Passage durch eine French-Druckzelle bei 8 bar aufgeschlossen. Der zellfreie Extrakt wurde in einen 1 l Erlenmeyerkolben gegeben, mit 1-Amino-1-desoxy-D-glucitol (Oxalatsalz) zu einer Konzentration von 10 g/l versetzt und bei 30°C auf einer Rundschüttelmaschine bei 280 Upm inkubiert. Nach 7 Stunden konnten in diesem Extrakt 3,7 g/l 6-Amino-6-desoxy-L-sorbose — entsprechend einer 37%igen Umsetzung — anhand der Saccharase-Hemmaktivität nachgewiesen werden. Die Umsetzung wurde zu diesem Zeitpunkt abgebrochen und der Extrakt wie in Beispiel 1 angegeben aufgearbeitet.

Beispiel 7

Oxidation von 1-Amino-1-desoxy-D-glucitol in einer definierten Enzymreaktion in einem zellfreien Extrakt von Glucobacter oxydans ssp. suboxydans

Ein zellfreier Extrakt von Glucobacter oxydans spp. suboxydans wurde wie in Beispiel 6 angegeben hergestellt. Dem Extakt wurden zugegeben:

NADP (Na-Salz) zu einer Konzentration von 0,2 mM als NADP-regenerierendes System eine mikrosomale Rohfraktion aus Saccharomyces cerevisiae (1 mg/ml) und 1-Amino-1-desoxy-D-glucitol (Oxalatsalz zu einer Konzentration von 10 g/l.

Das Gemisch wurde bei 30°C im Wasserbad inkubiert und mit Sauerstoff durchblasen. Die Bildung von 6-Amino-6-desoxy-L-sorbose wurde anhand der Zunahme der Saccharase-Hemmaktivität gemessen. Nach 7 Stunden war ein Gehalt von 3,7 g 6-Amino-6-desoxy-L-sorbose pro 1 l nachweisbar — entsprechend einer 37%igen Umsetzung —. Zu diesem Zeitpunkt wurde die Reaktion abgebrochen und das Gemisch wie in Beispiel 1 angegeben aufgearbeitet.

**Patentansprüche**

1. Verfahren zur Herstellung von 6-Amino-6-desoxy-L-sorbose, dadurch gekennzeichnet, daß man 1-Amino-1-desoxy-D-glucitol mit Sauerstoff unter Einwirkung von geeigneten aeroben oder fakultativ aeroben Mikroorganismen oder von deren Extrakten oxidiert.

2. Verfahren nach Anspruch 1, dadurch gekennzeichnet, daß man als aerobe Mikroorganismen solche der Ordnung Pseudomonadales verwendet.

3. Verfahren nach Anspruch 1, dadurch gekennzeichnet, daß man als aerobe Mikroorganismen coryneforme Bakterien verwendet.

4. Verfahren nach Anspruch 1, dadurch gekennzeichnet, daß man als aerobe Mikroorganismen Hefen der Ordnung Endomycetales verwendet.

5. Verfahren nach Anspruch 1, dadurch gekennzeichnet, daß man als Mikroorganismen Gluconobacter oxidans ssp. suboxidans (DSM 50 049) oder Glucobacter oxidans ssp. suboxidans (DSM 2003) einsetzt.

6. Verfahren nach Anspruch 1, dadurch gekennzeichnet, daß man als Mikroorganismus Cornynebacterium betae (DSM 20 141) einsetzt.

7. Verfahren nach Anspruch 1, dadurch gekennzeichnet, daß man als Milkroorganismus Metschnikowia pulcherrima (ATCC 20 515) einsetzt.

8. Verfahren nach Anspruch 1, dadurch gekennzeichnet, daß man die in den Ansprüchen 5—7 genannten Mikroorganismen in Form konzentrierter Zellsuspensionen einsetzt.

9. Verfahren nach Anspruch 1, dadurch gekennzeichnet, daß man nicht-fraktionierte Rohextrakte einsetzt, die aus den in den Ansprüche 5—7 genannten Mikroorganismen gewonnen wurden.

10. Verfahren nach Anspruch 1, dadurch gekennzeichnet, daß man gereinigte Extraktfraktionen einsetzt, die aus den in den Ansprüchen 5—7 genannten Mikroorganismen gewonnen wurden.

**Revendications**

1. Procédé pour la fabrication de 6-amino-6-désoxy-L-sorbose, caractérisé en ce que l'on oxyde de 1-amino-1-désoxy-D-glucitol par l'oxygène sous l'action de micro-organismes aérobies ou facultativement aérobies appropriés ou de leurs extraits.

2. Procédé selon la revendication 1, caractérisé en ce que l'on utilise comme micro-organismes ceux du genre des Pseudomonadales.

3. Procédé selon la revendication 1, caractérisé en ce que l'on utilise comme micro-organismes aérobies des bactéries corynéformes.

4. Procédé selon la revendication 1, caractérisé en ce que l'on utilise comme micro-organismes aérobies des levures de l'ordre des Endomycetales.

5. Procédé selon la revendication 1, caractérisé en ce que l'on utilise comme micro-organismes Gluconobacter oxydans ssp. suboxydans (DSM 50 049) ou Glucobacter oxydans ssp. suboxydans (DSM 2003).

6. Procédé selon la revendication 1, caractérisé en ce que l'on utilise comme micro-organisme Corynebacterium betae (DSM 20 141).

7. Procédé selon la revendication 1, caractérisé en ce que l'on utilise, comme micro-organisme Metschnikowia pulcherrima (ATCC 20 515).

8. Procédé selon la revendication 1, caractérisé en ce que l'on utilise les micro-organismes mentionnés dans les revendications 5 à 7 sous forme de suspensions cellulaires concentrées.

9. Procédé selon la revendication 1, caractérisé en ce que l'on utilise des extraits bruts non fractionnés qui ont été obtenus à partir des micro-organismes mentionnés aux revendications 1 à 7.

10. Procédé selon la revendication 1, caractérisé en ce que l'on utilise des fractions d'extraits purifiées qui ont été obtenues à partir des micro-organismes mentionnés dans les revendications 5 à 7.

## Claims

1. Process for the preparation of 6-amino-6-deoxy-L-sorbose, characterised in that 1-amino-1-deoxy-D-glucitol is oxidised with oxygen accompanied by the action of suitable aerobic or facultatively aerobic micro-organisms or of extracts thereof.

2. Process according to Claim 1, characterised in that the aerobic micro-organisms used are those of the order Pseudomonadales.

3. Process according to Claim 1, characterised in that the aerobic micro-organisms used are corneforme bacteria.

4. Process according to Claim 1, characterised in that yeasts of the order Endomycetales are used as the aerobic micro-organisms.

5. Process according to Claim 1, characterised in that the micro-organisms used are Gluconobacter oxidans ssp. suboxidans (DSM 50 049) or Glucobacter oxidans ssp. suboxidans (DSM 2003).

6. Process according to Claim 1, characterised in that Cornynebacterium betae (DSM 20 141) is used as the micro-organism.

7. Process according to Claim 1, characterised in that Metschnikowia pulcherrima (ATCC 20 515) is used as the micro-organism.

8. Process according to Claim 1, characterised in that the micro-organisms named in Claims 5—7 are used in the form of concentrated cell suspensions.

9. Process according to Claim 1, characterised in that non-fractionated crude extracts are used which have been obtained from the micro-organisms named in Claims 5—7.

10. Process according to Claim 1, characterised in that purified extract fractions are used which have been obtained from the micro-organisms named in Claims 5—7.